(19) Europäisches Patentamt
European Patent Office
Office européen des brevets

(11) **EP 2 154 254 B1**

(12) **FASCICULE DE BREVET EUROPEEN**

(45) Date de publication et mention
de la délivrance du brevet:
**12.09.2012   Bulletin 2012/37**

(51) Int Cl.:
***C12Q 1/64*** *(2006.01)*

(21) Numéro de dépôt: **09290583.5**

(22) Date de dépôt: **23.07.2009**

(54) **Procédé pour prédire les changements de composition chimique d'huile d'un réservoir suite à une biodégradation**

Verfahren zur Vorraussage der Veränderungen chemischer Ölzusammensetzungen in einem Behälter infolge einer Biodegradation

Method for predicting changes in the chemical composition of the oil in a reservoir following biodegradation

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **01.08.2008   FR 0804435**

(43) Date de publication de la demande:
**17.02.2010   Bulletin 2010/07**

(73) Titulaire: **IFP Energies nouvelles**
**92852 Rueil-Malmaison Cedex (FR)**

(72) Inventeurs:
• **Haeseler, Frank**
**92250 La Garenne Colombes (FR)**
• **Behar, Françoise**
**75007 Paris (FR)**

(56) Documents cités:
**FR-A- 2 830 646     FR-A- 2 888 251**

• **CARPENTIER: "New concepts for biodegradation evaluation in oil fields, a combined geological and numerical approach", AAPG ANNUAL MEETING, XX, XX, 10 mars 2002 (2002-03-10), page 27, XP002376026,**
• **HEAD I M ET AL: "Biological activity in the deep subsurface and the origin of heavy oil", NATURE, NATURE PUBLISHING GROUP, LONDON, UK, vol. 426, no. 20, 1 janvier 2003 (2003-01-01), pages 344-352, XP002376027, ISSN: 0028-0836**
• **ROLING W F M ET AL: "The microbiology of hydrocarbon degradation in subsurface petroleum reservoirs: Perspectives and prospects", RESEARCH IN MICROBIOLOGY 200306 FR, vol. 154, no. 5, juin 2003 (2003-06), pages 321-328, XP002515693, ISSN: 0923-2508**

## Description

La présente invention concerne le domaine de l'industrie pétrolière. Elle concerne une méthode pour évaluer la biodégradation d'hydrocarbures présents dans une structure géologique, telle qu'un gisement pétrolier, suite à l'action d'une population bactérienne.

La méthode selon l'invention fournit un outil d'évaluation très utile notamment aux géologues soucieux d'orienter les investigations hors de zones à risque. En particulier, on peut l'utiliser dans le domaine technique de la modélisation de bassin pour prédire la quantité et la qualité de l'huile que l'on peut s'attendre à trouver en relation avec l'altération biologique qui peut advenir dans de tels environnements.

Un des problèmes couramment rencontrés lors de la définition de l'intérêt d'un objectif pétrolier, c'est-à-dire un piège à hydrocarbures non foré, situé à une relativement faible température (habituellement à moins de 80°C) est l'évaluation du risque de "biodégradation".

En effet il est couramment reconnu que la biodégradation, définie comme la destruction sélective d'une partie des molécules composant un brut pétrolier par des bactéries, peut se développer jusqu'à des températures pouvant atteindre 70 à 80°C. De telles températures sont courantes en particulier dans les sédiments marins qui sont des zones où la recherche pétrolière est la plus active actuellement.

Cette biodégradation a pour effet de modifier la qualité de l'huile. Cette qualité est mesurée par la viscosité et le degré API qui est inversement proportionnel à la densité. Elle est fonction de la composition de l'huile (l'échelle de degrés API est entre 0 et 40). Une forte teneur en hydrocarbures légers, augmente le degré API, alors qu'une forte proportion de résines et d'asphaltènes (NSO) fait décroître le degré API. Au cours de l'histoire géologique d'un fluide, la maturation thermique tend à alléger les huiles de réservoir, donc tend à augmenter le degré API, tandis que l'altération microbienne tend à alourdir l'huile, et donc à décroître son degré API (Conan et al. 1979 Advances in Organic Geochemistry, Pegamon Press Oxford 1-17 et Peters et Moldowan, 1993, The biomarker guide, Prentice Hall).

Un autre problème induit par la biodégradation des hydrocarbures est la production de gaz (méthane et gaz acides : $CO_2$ et $H_2S$). Certains de ces gaz ont une valeur économique (c'est le cas du méthane) et d'autres réduisent la valeur économique du réservoir ($H_2S$).

La biodégradation est un phénomène biologique qui peut affecter la quantité et la composition chimique des huiles de réservoir. Les bactéries, responsables de ces réactions, sont présentes dans le milieu poreux et vivent dans l'eau qui circule dans le milieu poreux. Ces bactéries utilisent les hydrocarbures comme source de carbone et d'énergie pour assurer leur développement et leur maintien. En faisant cela, d'une part elle dégradent sélectivement certains hydrocarbures et donc modifient à la fois la quantité et la composition de l'huile en place et d'autre part elles génèrent des métabolites comme des espèces gazeuses ($CH_4$, $H_2S$ et $CO_2$) et de nouvelles structures chimiques comme les acides carboxyliques. Néanmoins, les acides générés sont à leur tour dégradés et ne représentent en fait qu'une faible quantité dans les huiles biodégradées résiduelles. Pour faire vivre les bactéries, il faut également apporter au système des ingrédients comme les accepteurs d'électrons et les nutriments. Les réactions biologiques ont besoin de nutriments comme le phosphore et l'azote ainsi que des métaux, éléments indispensables à la synthèse des molécules constitutives des bactéries. Leur absence signifie l'arrêt de la croissance bactérienne.

Le processus de biodégradation peut être décrit de la façon suivante. Les microorganismes présents dans les milieux poreux utilisent la source d'énergie et de carbone que constituent certaines familles d'hydrocarbures présentes dans les huiles de réservoir avec deux objectifs :

- se développer et produire le plus possible de biomasse tant que l'azote et le phosphore ne sont pas épuisés et que les éléments en trace comme les métaux ne sont pas des facteurs limitants

- se maintenir, c'est à dire utiliser l'énergie disponible à travers des réactions de dégradation qui ne génèrent pas de biomasse supplémentaire. Durant ces réactions, les hydrocarbures sont effectivement dégradés et des métabolites sont générés mais aucun accroissement de biomasse n'est observé;

Ainsi, dans l'industrie pétrolière, il est très important de connaître le rôle de la biodégradation pour connaître la qualité et la quantité d'huile attendue dans un bassin sédimentaire. En effet, la biodégradation représente un risque majeur pour les compagnies pétrolières dont les forages en mer profonde représentent un investissement financier important. Toute méthode permettant de réduire ce risque est donc d'un intérêt majeur pour ces compagnies. Pour y parvenir, il faut disposer d'une méthode permettant d'estimer l'effet de la biodégradation sur la quantité et la qualité de l'huile.

## Présentation de l'art antérieur

Certains auteurs ont pu estimer des cinétiques de dégradation en corrélant des temps de séjour de l'huile dans le sous-sol avec le niveau de biodégradation (Larter et al 2003 Organic Geochemistry 4, 6001-613, Behar et al, 2006, Organic Geochemistry 37, 1042-1051 et de Barros Penteado et al, 2007, Organic Geochemistry 38, 1197-1211). Il s'agit là essentiellement d'un travail descriptif, visant à constater la biodégradation et ses conséquences, ainsi qu'à déterminer pour certains bassins des vitesses spécifiques de biodégradation. En particulier, les travaux de Larter permettent de prédire un taux de biodégradation sans tenir compte de la composition de l'huile, ce taux étant principalement appliqué aux hydrocarbures saturés. Par ailleurs, le moteur de la biodégradation est la diffusion des hydrocarbures au niveau de la zone de contact eau/huile dans la mesure ou c'est cette zone de contact qui fournit la source des accepteurs d'électrons.

Pour estimer de façon prédictive le niveau de biodégradation des huiles dans un bassin, on connaît une méthode décrite dans le brevet EP 1.436.412. Cette méthodologie est basée sur une analyse statistique du nombre de bactéries présentes dans le sous-sol en fonction de la profondeur. De même, on connaît du document FR 2 888 251 A, une méthode pour évaluer la biodégradation, à partir de données relatives aux caractéristiques physiques et géométriques de la structure étudiée : on détermine la masse d'hydrocarbures présente dans la structure sans tenir compte de la biodégradation ; puis on calcule la masse d'hydrocarbures consommée par biodégradation après avoir évalué le nombre de bactéries, leur consommation en hydrocarbure et le temps de remplissage de la structure ; on déduit de ces deux masses le pourcentage massique d'huile disparue par action bactérienne.

Par ailleurs, le résultat de ces approches n'est pas compositionnel.

De façon générale, les méthodes connues visent soit à décrire la biodégradation et non à la prédire, soit à prédire la biodégradation en prenant en compte le plan de contact eau/huile pour contrôler les vitesses de biodégradation. Ces types de méthodes ne tiennent pas compte des facteurs limitants l'activité des microorganismes : les accepteurs d'électrons. Ainsi, ces méthodes ne sont pas réellement prédictives et nécessitent un calage avec des données de puits.

Ainsi l'objet de l'invention concerne une méthode alternative pour évaluer l'évolution compositionnelle d'hydrocarbures piégés dans un milieu poreux suite à une biodégradation.

## La méthode selon l'invention

L'invention concerne une méthode pour déterminer une composition de fluides présents dans un milieu poreux suite à une biodégradation. Elle comporte les étapes suivantes :

- on définit un schéma réactionnel de la biodégradation, dans lequel lesdites classes chimiques réagissent avec des accepteur d'électrons, et dans lequel on impose que lesdites classes réagissant simultanément avec un premier accepteur d'électrons ayant le potentiel d'oxydoréduction le plus fort, selon des vitesses de réaction différentes et jusqu'à épuisement dudit premier accepteur dans le milieu, puis réagissent séquentiellement aux autres accepteurs d'électrons selon un même principe ;

- on détermine lesdites vitesses de réactions de chacune desdites classes chimiques en fonction d'au moins : - la concentration potentielle des classes chimiques dans l'eau ; - leur biodégradabilité intrinsèque ; - leur réactivité par rapport auxdits accepteurs d'électrons ; - la température du milieu ; et - le temps de séjour desdits fluides au sein du milieu ; et

- on détermine la composition desdits fluides en estimant une quantité de chacune desdites classes chimiques du schéma compositionnel en appliquant le schéma réactionnel.

Les accepteurs d'électrons peuvent être choisis parmi les accepteurs suivants : $O_2$, $NO_3$, $SO_4$ et $H_2O$.

Selon l'invention on peut définir les vitesses de réactions par un produit d'un premier terme ($R_t$) qui permet de prendre en compte un effet du temps de séjour dans le milieu poreux, un second terme ($R_T$) pour prendre en compte un effet de la température, et un troisième terme ($V_C$) pour prendre en compte l'accessibilité et la biodégradabilité intrinsèque de chacune des classes chimiques.

Le premier terme $R_t$ peut représenter un rendement de biodégradation et peut être déterminé au moyen d'une première courbe d'évolution du rendement $R_t$ en fonction du temps de séjour dans le milieu. Cette courbe peut être une fonction exponentielle du temps, où le rendement $R_t$ est supérieur à 95 % au-delà d'environ 2000 ans.

Le second terme $R_T$ peut représenter un rendement de biodégradation et peut être déterminé au moyen d'une courbe gaussienne d'évolution du rendement $R_T$ en fonction de la température dans le milieu. Cette courbe gaussienne est avantageusement centrée sur une température d'environ 30°C et s'annule pour 0°C et environ 70°C.

Le troisième terme ($V_C$) peut être déterminé en considérant que la vitesse de biodégradabilité intrinsèque des $C_6$-$C_{14}$ saturés est plus grande que celles des autres classes, et en considérant que la vitesse d'accessibilité maximale des $C_6$-$C_{14}$ aromatiques est plus grande que celles des autres classes.

Ainsi, les vitesses de réactions peuvent être définies par :

$$V\,C_{14}\text{-sat} \quad = \quad 0.4 \times V_{max}$$

$$V\,C_{14}\text{-aro} \quad = \quad 0.3 \times V_{max}$$

$$V\,C_{14+}n \quad = \quad 0.2 \times V_{max}$$

$$V\,C_{14+}\text{iso} \quad = \quad 0.18 \times V_{max}$$

$$V\,C_{14+}\text{cyclanes} \quad = \quad 0.08 \times V_{max}$$

$$V\,C_{14+}\text{aro} \quad = \quad 0.05 \times V_{max}$$

$$V\,NSO \quad = \quad 0$$

où $V_{max}$ est vitesse maximale de biodégradation définie par le produit de la vitesse maximale de biodégradabilité intrinsèque par la vitesse maximale d'accessibilité.

Selon un mode de réalisation, le milieu peut être un réservoir pétrolier, et on déduit de la composition des fluides une quantité de gaz acides produit lors de la biodégradation, pour déterminer des conditions d'exploitation du réservoir pétrolier.

Selon un autre mode de réalisation, le milieu peut être un réservoir pétrolier et on déduit de la composition des fluides une quantité de méthane produit lors de la biodégradation, pour déterminer des conditions d'exploitation du réservoir pétrolier.

## Présentation succincte des figures

D'autres caractéristiques et avantages de la méthode selon l'invention, apparaîtront à la lecture de la description ci-après d'exemples non limitatifs de réalisations, en se référant aux figures annexées et décrites ci-après.

La figure 1 illustre l'évolution, en fonction du temps t, du terme $R_t$, terme permettant de prendre en compte dans la vitesse globale de biodégradation l'effet du temps de séjour dans le milieu poreux.

La figure 2 illustre l'évolution, en fonction de la température T, du terme $R_T$, terme permettant de prendre en compte dans la vitesse globale de biodégradation l'effet de la température.

## Description détaillée de la méthode

La méthode selon l'invention permet d'évaluer la biodégradation d'hydrocarbures présents dans une structure géologique telle qu'un réservoir pétrolier. Il s'agit d'évaluer l'évolution de la quantité de composants chimiques contenus dans ces hydrocarbures, au cours de la migration des hydrocarbures et du remplissage de la structure (appelé piège). Cette évaluation permet, par exemple, de déterminer l'opportunité d'exploiter un réservoir pétrolier, et les conditions d'exploitation de ce réservoir.

La méthode comporte les étapes suivantes :

1. on définit un schéma compositionnel pour décrire les hydrocarbures et des gaz issus de la biodégradation ;

2. on estime la quantité de chaque classe chimique du schéma compositionnel, présente dans les hydrocarbures avant biodégradation ;

3. on définit un schéma réactionnel dans lequel les classes chimiques réagissent parallèlement avec des accepteurs d'électrons, et chacune des classes chimiques réagit séquentiellement avec les accepteurs d'électrons ;

4. on évalue l'évolution de la quantité de chaque classe chimique au moyen du schéma réactionnel.

1- Schéma compositionnel de la biodégradation

L'action bactérienne, à l'origine de la biodégradation des hydrocarbures, génère des gaz non hydrocarbures ($CO_2$, $H_2S$) et du méthane ($CH_4$). Elle altère plus vite les hydrocarbures saturés n et les hydrocarbures saturés iso. Ensuite les structures cycliques saturées et les aromatiques peuvent être touchés. En principe les composés soufrés et azotés (NSOs) restent inchangés, ainsi que les gaz $C_2$-$C_4$ sauf dans des conditions extrêmes. Ainsi, l'ordre à priori pour l'altération est le suivant : $C_6$-$C_{14}$ sat et aromatiques, $C_{14+}$ saturés.

Selon l'invention, on utilise les douze classes chimiques suivantes, afin de formaliser de façon compositionnelle la biodégradation :

| Classe 1 | $CO_2$ |
|---|---|
| Classe 2 | $H_2S$ |
| Classe 3 | $C_1$ |
| Classe 4 | $C_2$-$C_4$ |
| Classe 5 | $C_6$-$C_{14}$ saturés n et $C_6$-$C_{14}$ saturés iso |
| Classe 6 | $C_6$-$C_{14}$ saturés cyclo |
| Classe 7 | $C_6$-$C_{14}$ aromatiques |
| Classe 8 | $C_{14+}$ saturés n |
| Classe 9 | $C_{14+}$ saturés iso |
| Classe 10 | $C_{14+}$ saturés cyclo |
| Classe 11 | $C_{14+}$ aromatiques |
| Classe 12 | NSOs |

Avec la nomenclature classique suivante :

$C_1$ : famille d'hydrocarbures à 1 atome de carbone

$C_2$ : famille d'hydrocarbures à 2 atomes de carbone

$C_3$ : famille d'hydrocarbures à 3 atomes de carbone

$C_4$ : famille d'hydrocarbures à 4 atomes de carbone

$C_6$-$C_{14}$ ou $C_{14-}$ : hydrocarbures ayant entre 6 et 14 atomes de carbone

C14+ : hydrocarbures ayant plus de 14 atomes de carbone

NSOs : composés hétéroatomiques généralement de haute masse moléculaires (résines et asphaltènes) contenant des fonctions azotées, soufrées et oxygénées)

Les classes 3 à 12 permettent de décrire les hydrocarbures avant et après la biodégradation. Les classes 1 et 2 permettent de prendre en compte la génération de gaz non hydrocarbure pendant la biodégradation, ce qui est important pour déterminer les condition d'exploitation d'un champ.

2- Estimation compositionnelle avant biodégradation

Ce type d'estimation peut classiquement être réalisée à partir d'un outil, connu des spécialistes, appelé « simulateur de bassin », ou « modèle de bassin ». Un exemple de méthode utilisée par ce type d'outil logiciel est décrit dans la demande de brevet FR 2.906.482. Cette demande décrit une méthode de modélisation du craquage thermique du kérogène et des produits pétroliers associés.

3- Schéma réactionnel de biodégradation

L'unité de base pour les calculs de la biodégradation est la concentration molaire $C_{mi}$ des composés (classes chimiques) $i$ pris en compte dans le schéma compositionnel. Cette concentration molaire est définie par la formule présentée dans l'équation (1).

$$C_{mi} = \frac{\dfrac{C_i}{M_i}}{\dfrac{C_o}{M_o}} \qquad (1)$$

avec

$C_i$ : concentration massique du composé i

$M_i$ : masse molaire du composé i

$C_o$ : concentration massique de l'huile

$M_o$ : masse molaire de l'huile

La masse molaire de l'huile est un paramètre qui dépend des caractéristiques du pétrole. Sa valeur est fonction du type de kérogène ayant entraîné la genèse de l'huile, de l'histoire thermique du système pétrolier et en particulier du craquage secondaire que peut avoir subit le pétrole. La masse molaire de l'huile dépend également de la biodégradation, ainsi une huile biodégradée est elle plus lourde qu'elle ne l'était avant biodégradation.

$$M_o = f(\text{Type I, II, III}), f(\text{histoire thermique}), f(\text{biodégradation})$$

La minéralisation des hydrocarbures sous l'action de bactéries hydrocarbonoclastes conduit d'une part, à la disparition complète des hydrocarbures initialement présents et d'autre part, à la production des métabolites finaux suivants :

- $CO_2$ et $H_2O$ en conditions aérobies

- $CO_2$, $H_2O$ et $N_2$ en conditions dénitrifiantes

- $CO_2$, $H_2O$ et $H_2S$ en conditions sulfatoréductrices

- $CO_2$, et $CH_4$ en condition méthanogènes.

Ces équations décrivent les voies de biodégradation des hydrocarbures avec différents accepteurs d'électrons. Il s'agit de conditions de biodégradation aérobie (en présence d'oxygène), dénitrifiantes (en présence de nitrate), sulfato-réductrices (en présence de sulfate) et méthanogènes. Cette succession de mécanismes faisant appel à des accepteurs d'électrons ayant un potentiel d'oxydoréduction de plus en plus faible, ne peut se faire qu'après épuisement de l'accepteur d'électron précédent.

Dans les systèmes naturels, la biodégradation des hydrocarbures est partielle. D'une part des hydrocarbures résiduels subsistent toujours, au moins à l'état de traces, et d'autre part, des intermédiaires métaboliques, réfractaires à une biodégradation ultérieure, sont produits par les bactéries et peuvent s'accumuler.

Il est possible d'écrire les équations globales de biodégradation des hydrocarbures (CxHy) en faisant apparaître un terme de métabolites sous la forme d'un acide organique ou d'un di-ol, qui correspond à la formule chimique suivante :

CxH(y-2)O$_2$. Une réaction de ce type correspond donc à une minéralisation partielle, à une biodégradation incomplète, même quand l'ensemble des hydrocarbures disparaît. Les équations suivantes font abstraction de la formation de biomasse qui peut être une conséquence (non impérative) de la biodégradation.

L'énergie que les bactéries peuvent assimiler à partir du métabolisme de leur substrat dépend du potentiel d'oxydoréduction de l'accepteur d'électrons. Un potentiel d'oxydoréduction élevé (cas de l'oxygène et dans une moindre mesure du nitrate) va permettre un haut rendement énergétique, tandis qu'un faible potentiel d'oxydoréduction (cas du sulfate et de l'eau) s'accompagnera d'un rendement énergétique plus limité pour les bactéries impliquées. Il s'avère que les métabolites liposolubles s'accumulent quand le potentiel d'oxydoréduction des accepteurs d'électrons baisse. Ce phénomène est lié au rendement énergétique limité. Ainsi on constate que lors de la biodégradation des hydrocarbures dans l'environnement, la concentration en composés oxygénés présents dans la phase huile résiduelle a tendance à augmenter.

<u>3a- Équations séquentielles et globales de biodégradation</u>

Selon l'invention, le schéma réactionnel de biodégradation peut comporter un ensemble de réactions séquentielles pour chaque composé contenu dans l'hydrocarbure biodégradé. Il s'agit de réactions stoechiométriques séquentielles, qui présentent l'avantage de faire apparaître un métabolite $C_xH_{(y-2)}O_2$ avec une vitesse v1 et de le faire ensuite disparaître à une vitesse v2. Pour chaque réaction, la valeur absolue de v1 par rapport à celle de v2 permet de déterminer l'accumulation du métabolite $C_xH_{(y-2)}O_2$. Cette accumulation dépend également du rendement global des réactions qui, en fonction de certaines conditions peuvent être incomplètes. La vitesse V correspond à la réaction globale.

Les équations séquentielles de biodégradation d'un hydrocarbure de formule CxHy avec les principaux accepteurs d'électrons ($O_2$, $NO_3$, $SO_4$ et $H_2O$), sont présentées ci-après.

**accepteur d'électrons : $O_2$, biodégradation aérobie**

$$C_xH_y + 3/2\ O_2 \xrightarrow{v1_{O_2}} C_xH_{(y-2)}O_2 + H_2O$$

$$C_xH_{(y-2)}O_2 + (x+1/4y-3/2)\ O_2 \xrightarrow{v2_{O_2}} x\ CO_2 + (1/2y-1)\ H_2O$$

$$\underline{\text{Équation globale}} : C_xH_y + (x+1/4y)\ O_2 \xrightarrow{v_{O_2}} x\ CO_2 + 1/2y\ H_2O$$

$$\underline{\text{Exemple}} : C_{16}H_{34} + (49/2)\ O_2 \xrightarrow{v_{O_2}} 16\ CO_2 + 17\ H_2O$$

**accepteur d'électrons : $NO_3$, dénitrification**

$$C_xH_y + NO_3 \xrightarrow{v1_{NO_3^{2-}}} C_xH_{(y-2)}O_2 + 1/2\ N_2 + H_2O$$

$$C_xH_{(y-2)}O_2 + (2/3x+1/6y-1)\ NO_3 \xrightarrow{v2_{NO_3^{2-}}} x\ CO_2 + (1/3x+1/12y-1/2)\ N_2 + (1/2y-1)\ H_2O$$

$$\underline{\text{Équation globale}} : C_xH_y + (2/3x+1/6y)\ NO_3 \xrightarrow{v_{NO_3^{2-}}} x\ CO_2 + (1/3x+1/12y)\ N_2 + 1/2y\ H_2O$$

Exemple : $C_{16}H_{34} + (49/3) NO_3 \xrightarrow{V_{NO_3^{2-}}} 16 CO_2 + (49/6) N_2 + 17 H_2O$

accepteur d'électrons : SO$_4$, sulfatoréduction

$C_xH_y + 13/10 SO_4 \xrightarrow{V1_{SO_4^{2-}}} C_{(x-1)}H_{(y-5)}O_2 + CO_2 + 13/10 H_2S + 6/5 H_2O$

$C_{(x-1)}H_{(y-5)}O_2 + (2/5x+1/10y-13/10) SO_4 \xrightarrow{V2_{SO_4^{2-}}} (x-1) CO_2 + (2/5x+1/10y-13/10) H_2S + (-2/5x+2/5y-6/5) H_2O$

Équation globale : $C_xH_y + (2/5x+1/10y) SO_4 \xrightarrow{V_{SO_4^{2-}}} x CO_2 + (2/5x+1/10y) H_2S + (-2/5x+2/5y) H_2O$

Exemple : $C_{16}H_{34} + (98/10) SO_4 \xrightarrow{V_{SO_4^{2-}}} 16 CO_2 + (98/10) H_2S + (36/5) H_2O$

accepteur d'électrons : H$_2$O, méthanogénèse

$C_xH_y + 2 H_2O \xrightarrow{V1_{H_2O}} C_xH_{(y-2)}O_2 + 3 H_2$

$3 H_2 + 3/4 CO_2 + 3/4 H_2O \xrightarrow{V_{H_2}} 3/4 CH_4 + 9/4 H_2O$

$C_xH_{(y-2)}O_2 + (x-1/4y-1/2) H_2O \xrightarrow{V2_{H_2O}} (1/2x-1/8y-3/4) CO_2 + (1/2x+1/8y-3/4) CH_4$

Équation globale : $C_xH_y + (x-1/4y) H_2O \xrightarrow{V_{H_2O}} (1/2x-1/8y) CO_2 + (1/2x+1/8y) CH_4$

Exemple : $C_{16}H_{34} + (15/2) H_2O \xrightarrow{V_{H_2O}} (15/4) CO_2 + (49/4) CH_4$

Ainsi, le schéma réactionnel de biodégradation selon l'invention, est défini par l'ensemble des réactions chimiques suivantes :

- pour chaque classe chimique présentes dans les hydrocarbures, les réactions avec les accepteurs d'électrons (oxygène, nitrate, sulfate ou H$_2$O) sont séquentielles : chaque classe réagit avec l'accepteur d'électrons ayant le potentiel d'oxydoréduction le plus fort, jusqu'à épuisement de cet accepteur, puis réagit avec l'accepteur d'électrons restant dans le milieu et ayant le potentiel d'oxydoréduction le plus fort. Ce mécanisme peut se répéter jusqu'à épuisement des accepteurs d'électrons.

- les classes chimiques réagissent en parallèle : il n'existe pas de mécanisme séquentiel, dans lequel une classe chimique réagirait une fois qu'une autre classe aurait fini de réagir. En fait, toutes les classes peuvent réagir en même temps, mais avec des cinétiques différentes.

Pour définir complètement ce schéma réactionnel, il faut donc définir les différentes vitesses de réactions (V1 et V2 pour les réaction séquentielles, ou V pour la réaction globale) pour chaque classe chimique.

3b- Expressions des vitesses de biodégradation

L'équation de vitesse de biodégradation peut s'écrire ainsi :

$$V_i = -\frac{\partial C_{mi}}{\partial t} = -\frac{C_{mi}(t_n) - C_{mi}(t_{n-1})}{t_n - t_{n-1}}$$

avec

$C_{mi}$ : concentration molaire du composé i qui fait l'objet de la biodégradation

$C_{mi}(t_n)$ : concentration molaire du composé i au temps $t_n$

$C_{mi}(t_{n-1})$ : concentration molaire du composé i au temps $t_{n-1}$

Cette vitesse $V_i$ est entre autre fonction de l'accepteur d'électron, de la biomasse, du composé i, de la température T, de l'hydrodynamique des fluides et de la diffusion.
Les accepteurs d'électron sont : $O_2$, $NO_3$, $SO_4$, $H_2O$. Ce paramètre dépend du type de réservoir. La biomasse concerne les éléments nutritifs (N, P, K) et les éléments traces (Fe, Mg, Co...). Ce paramètre dépend également du type de réservoir. Le composé i est une des classes chimiques définies dans le schéma compositionnel de l'étape 1. La température T intervient à un instant donné (température au moment de la réaction), mais également en fonction de l'histoire thermique (paléostérilisation). L'hydrodynamique concerne le déplacement de l'huile dans le réservoir et/ou dans les chemins de migration, ainsi que l'évolution du contact huile/eau, et du flux d'eau.
Selon, les accepteurs d'électron, on peut ainsi écrire :
**$O_2$**

Pour l'équation $C_xH_y + O_2 \xrightarrow{v1} C_xH_yO_2,$ on peut écrire :

$$V1(O_2)_{C_6-C_{14}sat} = \frac{\partial C_{mC_xH_y}}{\partial t} = \frac{C_{mC_xH_y}(t_n) - C_{mC_xH_y}(t_{n-1})}{t_n - t_{n-1}}$$

$$V1(O_2)_{C_6-C_{14}sat} = \frac{\partial C_{mO_2}}{\partial t} = \frac{C_{mO_2}(t_n) - C_{mO_2}(t_{n-1})}{t_n - t_{n-1}}$$

$$V1(O_2)_{C_6-C_{14}sat} = -\frac{\partial C_{mC_xH_yO_2}}{\partial t} = -\frac{C_{mC_xH_yO_2}(t_n) - C_{mC_xH_yO_2}(t_{n-1})}{t_n - t_{n-1}}$$

Et pour l'équation $C_xH_yO_2 + (x+1/4y-1)\, O_2 \xrightarrow{r2} xCO_2 + 1/2x\ H_2O,$ on peut écrire :

$$V2(O_2)_{C_6-C_{14}sat} = \frac{\partial C_{mC_xH_yO_2}}{\partial t} = \frac{C_{mC_xH_yO_2}(t_n) - C_{mC_xH_yO_2}(t_{n-1})}{t_n - t_{n-1}}$$

$$V2(O_2)_{C_6-C_{14}sat} = \frac{\partial C_{mO_2}}{\partial t} = \frac{C_{mO_2}(t_n) - C_{mO_2}(t_{n-1})}{t_n - t_{n-1}}$$

$$V2(O_2)_{C_6-C_{14}sat} = -\frac{\partial C_{mCO_2}}{\partial t} = -\frac{C_{mCO_2}(t_n) - C_{mCO_2}(t_{n-1})}{t_n - t_{n-1}}$$

$$V2(O_2)_{C_6-C_{14}sat} = -\frac{\partial C_{mH_2O}}{\partial t} = -\frac{C_{mH_2O}(t_n) - C_{mH_2O}(t_{n-1})}{t_n - t_{n-1}}$$

**NO$_3$**

Pour l'équation $C_xH_y + 2/3\ NO_3 \xrightarrow{r1} C_xH_yO_2 + 4/3\ N_2$ on peut écrire :

$$V1(NO_3)_{C_6-C_{14}sat} = \frac{\partial C_{mC_xH_y}}{\partial t} = \frac{C_{mC_xH_y}(t_n) - C_{mC_xH_y}(t_{n-1})}{t_n - t_{n-1}}$$

$$V1(NO_3)_{C_6-C_{14}sat} = \frac{\partial C_{mNO_3}}{\partial t} = \frac{C_{mNO_3}(t_n) - C_{mNO_3}(t_{n-1})}{t_n - t_{n-1}}$$

$$V1(NO_3)_{C_6-C_{14}sat} = -\frac{\partial C_{mC_xH_yO_2}}{\partial t} = -\frac{C_{mC_xH_yO_2}(t_n) - C_{mC_xH_yO_2}(t_{n-1})}{t_n - t_{n-1}}$$

$$V1(NO_3)_{C_6-C_{14}sat} = -\frac{\partial C_{mN_2}}{\partial t} = -\frac{C_{mN_2}(t_n) - C_{mN_2}(t_{n-1})}{t_n - t_{n-1}}$$

Et pour l'équation $C_xH_yO_2 + (2/3x+1/6y-2/3)\ NO_3 \xrightarrow{r2} x\ CO_2 + 1/2y\ H_2O + (1/3x+1/12y-1/3)\ N_2$ on peut écrire :

$$V2(NO_3)_{C_6-C_{14}sat} = \frac{\partial C_{mC_xH_yO_2}}{\partial t} = \frac{C_{mC_xH_yO_2}(t_n) - C_{mC_xH_yO_2}(t_{n-1})}{t_n - t_{n-1}}$$

$$V2(NO_3)_{C_6-C_{14}sat} = \frac{\partial C_{mNO_3}}{\partial t} = \frac{C_{mNO_3}(t_n) - C_{mNO_3}(t_{n-1})}{t_n - t_{n-1}}$$

$$V2(NO_3)_{C_6-C_{14}sat} = -\frac{\partial C_{mCO_2}}{\partial t} = -\frac{C_{mCO_2}(t_n) - C_{mCO_2}(t_{n-1})}{t_n - t_{n-1}}$$

$$V2(NO_3)_{C_6-C_{14}sat} = -\frac{\partial C_{mH_2O}}{\partial t} = -\frac{C_{mH_2O}(t_n) - C_{mH_2O}(t_{n-1})}{t_n - t_{n-1}}$$

$$V2(NO_3)_{C_6-C_{14}sat} = -\frac{\partial C_{mN_2}}{\partial t} = -\frac{C_{mN_2}(t_n) - C_{mN_2}(t_{n-1})}{t_n - t_{n-1}}$$

**SO$_4$**

Pour l'équation $C_xH_y + 5/4\ H_2O + 5/4\ SO_4 \xrightarrow{V1} C_{(x-1)}H_yO_2 + CO_2 + 5/4\ H_2S,$ on peut écrire :

$$V1(SO_4)_{C_6-C_{14}sat} = \frac{\partial C_{mC_xH_y}}{\partial t} = \frac{C_{mC_xH_y}(t_n) - C_{mC_xH_y}(t_{n-1})}{t_n - t_{n-1}}$$

$$V1(SO_4)_{C_6-C_{14}sat} = \frac{\partial C_{mSO_4}}{\partial t} = \frac{C_{mSO_4}(t_n) - C_{mSO_4}(t_{n-1})}{t_n - t_{n-1}}$$

$$V1(SO_4)_{C_6-C_{14}sat} = \frac{\partial C_{mH_2O}}{\partial t} = \frac{C_{mH_2O}(t_n) - C_{mH_2O}(t_{n-1})}{t_n - t_{n-1}}$$

$$V1(SO_4)_{C_6-C_{14}sat} = -\frac{\partial C_{mC_xH_yO_2}}{\partial t} = -\frac{C_{mC_xH_yO_2}(t_n) - C_{mC_xH_yO_2}(t_{n-1})}{t_n - t_{n-1}}$$

$$V1(SO_4)_{C_6-C_{14}sat} = -\frac{\partial C_{mCO_2}}{\partial t} = -\frac{C_{mCO_2}(t_n) - C_{mCO_2}(t_{n-1})}{t_n - t_{n-1}}$$

$$V1(SO_4)_{C_6-C_{14}sat} = -\frac{\partial C_{mH_2S}}{\partial t} = -\frac{C_{mH_2S}(t_n) - C_{mH_2S}(t_{n-1})}{t_n - t_{n-1}}$$

Et pour l'équation $C_{(x-1)}H_yO_2 + (2/5x+1/10y-2/5)\ SO_4 \rightarrow (x-1)\ CO_2 + (y-x+1)\ H_2O + (2/5x+1/10y-2/5)\ H_2S,$ on

peut écrire :

$$V2(SO_4)_{C_6-C_{14}sat} = \frac{\partial C_{mC_xH_yO_2}}{\partial t} = \frac{C_{mC_xH_yO_2}(t_n) - C_{mC_xH_yO_2}(t_{n-1})}{t_n - t_{n-1}}$$

$$V2(SO_4)_{C_6-C_{14}sat} = \frac{\partial C_{mSO_4}}{\partial t} = \frac{C_{mSO_4}(t_n) - C_{mSO_4}(t_{n-1})}{t_n - t_{n-1}}$$

$$V2(SO_4)_{C_6-C_{14}sat} = -\frac{\partial C_{mCO_2}}{\partial t} = -\frac{C_{mCO_2}(t_n) - C_{mCO_2}(t_{n-1})}{t_n - t_{n-1}}$$

$$V2(SO_4)_{C_6-C_{14}sat} = -\frac{\partial C_{mH_2O}}{\partial t} = -\frac{C_{mH_2O}(t_n) - C_{mH_2O}(t_{n-1})}{t_n - t_{n-1}}$$

$$V2(SO_4)_{C_6-C_{14}sat} = -\frac{\partial C_{mH_2S}}{\partial t} = -\frac{C_{mH_2S}(t_n) - C_{mH_2S}(t_{n-1})}{t_n - t_{n-1}}$$

**CH$_4$**

Pour l'équation $C_xH_y + 2\,H_2O \xrightarrow{V1} C_xH_yO_2 + H_2$, on peut écrire :

$$V1(CH_4)_{C_6-C_{14}sat} = \frac{\partial C_{mC_xH_y}}{\partial t} = \frac{C_{mC_xH_y}(t_n) - C_{mC_xH_y}(t_{n-1})}{t_n - t_{n-1}}$$

$$V1(CH_4)_{C_6-C_{14}sat} = \frac{\partial C_{mH_2O}}{\partial t} = \frac{C_{mH_2O}(t_n) - C_{mH_2O}(t_{n-1})}{t_n - t_{n-1}}$$

$$V1(CH_4)_{C_6-C_{14}sat} = -\frac{\partial C_{mC_xH_yO_2}}{\partial t} = -\frac{C_{mC_xH_yO_2}(t_n) - C_{mC_xH_yO_2}(t_{n-1})}{t_n - t_{n-1}}$$

$$V1(CH_4)_{C_6-C_{14}sat} = -\frac{\partial C_{mH_2}}{\partial t} = \frac{C_{mH_2}(t_n) - C_{mH_2}(t_{n-1})}{t_n - t_{n-1}}$$

Et pour l'équation $C_xH_yO_2 + 2/3(1-y)\,H_2O \xrightarrow{V2} 1/3(4-y)\,CO_2 + (x+1/3y-4/3)\,CH_4$, on peut écrire:

$$V2(CH_4)_{C_6-C_{14}sat} = \frac{\partial C_{mC_xH_yO_2}}{\partial t} = \frac{C_{mC_xH_yO_2}(t_n) - C_{mC_xH_yO_2}(t_{n-1})}{t_n - t_{n-1}}$$

$$V2(CH_4)_{C_6-C_{14}sat} = \frac{\partial C_{mH_2O}}{\partial t} = \frac{C_{mH_2O}(t_n) - C_{mH_2O}(t_{n-1})}{t_n - t_{n-1}}$$

$$V2(CH_4)_{C_6-C_{14}sat} = -\frac{\partial C_{mCO_2}}{\partial t} = -\frac{C_{mCO_2}(t_n) - C_{mCO_2}(t_{n-1})}{t_n - t_{n-1}}$$

$$V2(CH_4)_{C_6-C_{14}sat} = -\frac{\partial C_{mCH_4}}{\partial t} = -\frac{C_{mCH_4}(t_n) - C_{mCH_4}(t_{n-1})}{t_n - t_{n-1}}$$

Les équations précédentes sont écrites pour $C_6$-$C_{14}$ sat, mais elles sont rigoureusement identiques pour les autres pseudo-composants $C_6$-$C_{14}$ aro, $C_{14}$+ sat et $C_{14}$+ aro.

3c- Détermination des vitesses globales de biodégradation

Selon l'invention, on utilise préférentiellement les réactions globales. On affecte à chacune d'elle une vitesse, selon la classe chimique et l'accepteur d'électrons. Ces vitesses de réactions, sont appelées vitesses globales.

Ainsi, on détermine les vitesses globales de chacune des classes chimiques du schéma compositionnel, en fonction d'une part de l'accessibilité des hydrocarbures aux bactéries, c'est à dire leur concentration potentiellement soluble dans l'eau, et d'autre part, de leur biodégradabilité intrinsèque (capacité des microorganismes à les dégrader).

On considère qu'une telle vitesse globale est le produit de trois termes : un premier terme ($R_t$) qui permet de prendre en compte l'effet du temps de séjour dans le milieu poreux, un second terme ($R_T$) pour prendre en compte l'effet de la température, et un troisième terme ($V_C$) pour prendre en compte les spécificités de chacune des classes chimiques.

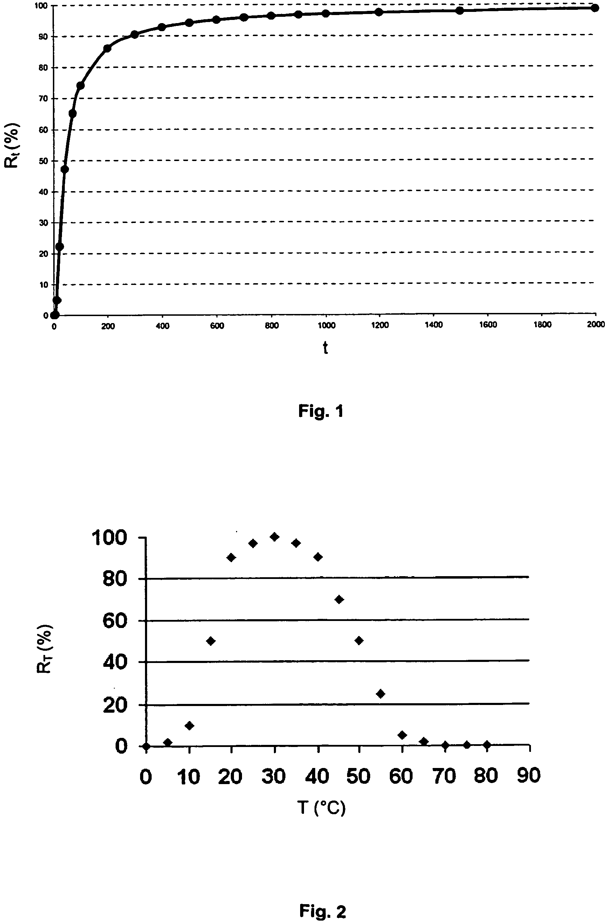

Le premier terme ($R_t$) permettant de prendre en compte l'effet du temps de séjour dans le milieu poreux représente un rendement de biodégradation. Il peut être déterminé à partir de la courbe représentés sur la figure 1, qui illustre l'évolution du rendement de biodégradation $R_t$ (%) en fonction du temps $t$ (en année). Dans cette figure on définit un temps plateau (ici de 2.000 ans) au delà duquel le rendement est supérieur à 95 % et en de ça duquel le rendement sera d'autant plus faible que le temps diminue.

Le second terme ($R_T$) permettant de prendre en compte l'effet de la température représente un rendement global de biodégradation. Il peut être déterminé en considérant que le rendement global $R_T$ évolue en fonction de la température selon une courbe gaussienne. Selon un exemple, illustré sur la figure 2, cette courbe gaussienne est centrée sur une température d'environ 30°C et s'annule pour 0°C et environ 70°C.

Le troisième terme ($V_C$) permettant de prendre en compte les spécificités de chacune des classes chimiques est composé de deux termes. Un premier rend compte de la biodégradabilité intrinsèque des différentes classes de composés. Si $V_{bio}$ est la vitesse globale maximum de biodégradabilité, on peut classer la biodégradabilité des différentes classes chimiques d'hydrocarbures comme suit :

$$(V_{bio})_{C14\text{-}sat} = (V_{bio})_{max}$$

$$(V_{bio})_{C14\text{-}aro} = 0.6 \times (V_{bio})_{max}$$

$$(V_{bio})_{C14+n} = (V_{bio})_{max}$$

$$(V_{bio})_{C14+iso} = (0.8) \times (V_{bio})_{max}$$

$$(V_{bio})_{C14+cyclanes} = (0.3) \times (V_{bio})_{max}$$

$$(V_{bio})_{C14+aro} = (0.1) \times (V_{bio})_{max}$$

$$(V_{bio})_{C14+nso} = (0.0) \times (V_{bio})_{max}$$

Le second terme permettant de définir $V_C$ rend compte de l'accessibilité des hydrocarbures dans le milieu poreux, c'est à dire leur capacité à être accessibles aux bactéries (par exemple les aromatiques $C_6$-$C_{14}$ sont les hydrocarbures les plus solubles dans l'eau, c'est donc eux qui auront l'accessibilité maximale).

$$(V_{accès})_{C14-aro} = (V_{accès})_{max}$$

$$(V_{accès})_{C14-sat} = 0.4 \times (V_{accès})_{max}$$

$$(V_{accès})_{C14+chains} = (0.2) \times (V_{accès})_{max}$$

$$(V_{accès})_{C14+cyclanes} = (0.2) \times (V_{accès})_{max}$$

$$(V_{accès})_{C14+aro} = (0.3) \times (V_{accès})_{max}$$

$$(V_{accès})_{C14+nso} = (0.0) \times (V_{accès})_{max}$$

Ainsi, le second terme $V_C$ pour les différentes classes chimiques est :

$$V_C\ C_{14}\text{-sat} \quad = \quad (V_{bio})max \quad\quad \times \quad 0.4 \times (V_{accès})max$$

$$V_C\ C_{14}\text{-aro} \quad = \quad 0.3 \times (V_{bio})max \quad\quad \times \quad (V_{accès})max$$

$$V_C \, C_{14+}n \quad = \quad (V_{bio})max \quad \quad x \quad 0.3) \, x \, (V_{accès})max$$

$$V_C \, C_{14+}iso \quad = \quad 0.7 \, x \, (V_{bio})max \quad \quad x \quad 0.2x \, (V_{accès})max$$

$$V_C \, C_{14+}cyclanes \quad = \quad 0.4 \, x \, (V_{bio})max \quad \quad x \quad 0.2 \, x \, (V_{accès})max$$

$$V_C \, C_{14+}aro \quad = \quad 0.15 \, x \, (V_{bio})max \quad \quad x \quad 0.3 \, x \, (V_{accès})max$$

$$V_C \, NSO \quad = \quad 0$$

Ainsi, en prenant en compte l'ensemble des termes ($V_{max} = (V_{bio})max \, x \, (V_{accès})max$), les vitesses globales (V) de biodégradation pour les différentes classes chimiques sont donc les suivantes

$$V \, C_{14}sat \quad = \quad (V_{bio})max \quad \quad x \quad 0.4 \, x \, (V_{accès})max \quad = \quad 0.4xV_{max}$$

$$V \, C_{14}aro \quad = \quad 0.3 \, x \, (V_{bio})max \quad \quad x \quad (V_{accès})max \quad = \quad 0.3xV_{max}$$

$$V \, C_{14+}n \quad = \quad (V_{bio})max \quad \quad x \quad 0.3) \, x \, (V_{accès})max \quad = \quad 0.2xV_{max}$$

$$V \, C_{14+}iso \quad = \quad (0.7) \, x \, (V_{bio})max \quad \quad x \quad 0.2x \, (V_{accès})max \quad = \quad 0.18xV_{max}$$

$$V \, C_{14+}cyclanes \quad = \quad (0.4) \, x \, (V_{bio})max \quad \quad x \quad (0.2) \, x \, (V_{accès})max \quad = \quad 0.08xV_{max}$$

$$V \, C_{14+}aro \quad = \quad (0.15) \, x \, (V_{bio})max \quad \quad x \quad (0.3) \, x \, (V_{accès})max \quad = \quad 0.05xV_{max}$$

$$V \, NSO \quad = \quad 0$$

4- Estimation de l'évolution de la composition de l'hydrocarbure

En appliquant le schéma réactionnel défini à l'étape 3, aux quantités de chaque classe chimique estimées à l'étape 2, on détermine les quantités de chaque classe chimique après biodégradation.

**Variantes / Applications**

Le domaine principal d'application est la prédiction de la qualité de l'huile suite à une altération biologique dans des modèles de bassin. Ainsi selon un mode de réalisation, on détermine le degré API et la viscosité des hydrocarbures ainsi dégradés au moyen de corrélations entre le degré API et la décroissance en hydrocarbures saturés ou en fonction de la concentration en composés NSOs.

Selon un autre mode de réalisation, on détermine la production des gaz d'origine biologique associés ($CH_4$,

$CO_2$ et $H_2S$) et à terme l'acidité des bruts. La méthode contribue ainsi à une meilleure évaluation économique du réservoir pétrolier, et permet donc de décider des forages.

Selon un autre mode de réalisation, les schémas compositionnel et réactionnel peuvent également s'appliquer à l'estimation de la biodégradation d'une huile en cours de production (échelle du réservoir).

Ces trois évaluations (qualité de l'huile, production de gaz acides ou de méthane, biodégradation encours de production) permettent chacune de déterminer l'opportunité d'exploiter un réservoir pétrolier, et si exploitation il y a, de déterminer les conditions d'exploitation (emplacement de forages, pressions des boues, matériel de forage, ...).

Selon un autre mode de réalisation les schémas compositionnel et réactionnel peuvent également s'appliquer à l'évaluation de la biodégradation des hydrocarbures dans les sites pollués.

Ainsi la méthode permet de déterminer la composition de fluides présents dans un milieu poreux suite à une biodégradation. Ces fluides correspondent d'une part aux hydrocarbures biodégradés, et d'autres part aux métabolites produits lors de cette biodégradation, et notamment les gaz d'origine biologique associés : $CH_4$, $CO_2$ et $H_2S$.

## Revendications

1. Méthode pour déterminer une composition de fluides présents dans un milieu poreux suite à une biodégradation, **caractérisé en ce que** :

   - on définit un schéma compositionnel de biodégradation à partir d'au moins les classes chimiques suivantes : $CO_2$, $H_2S$, $C_1$, $C_2$-$C_4$, $C_6$-$C_{14}$ saturés n et $C_6$-$C_{14}$ saturés iso, $C_6$-$C_{14}$ saturés cyclo, $C_6$-$C_{14}$ aromatiques, $C_{14}$, saturés n, $C_{14}$, saturés iso, $C_{14+}$ saturés cyclo, $C_{14+}$ aromatiques, NSOs ;
   - on détermine une quantité de chacune desdites classes chimiques contenues dans lesdits fluides avant biodégradation ;
   - on définit un schéma réactionnel de la biodégradation, dans lequel lesdites classes chimiques réagissent avec des accepteur d'électrons, et dans lequel on impose que lesdites classes réagissant simultanément avec un premier accepteur d'électrons ayant le potentiel d'oxydoréduction le plus fort, selon des vitesses de réaction différentes et jusqu'à épuisement dudit premier accepteur dans le milieu, puis réagissent séquentiellement aux autres accepteurs d'électrons selon un même principe ;
   - on détermine lesdites vitesses de réactions de chacune desdites classes chimiques en fonction d'au moins : - la concentration potentielle des classes chimiques dans l'eau ; - leur biodégradabilité intrinsèque ; - leur réactivité par rapport auxdits accepteurs d'électrons ; - la température du milieu ; et - le temps de séjour desdits fluides au sein du milieu ; et
   - on détermine la composition desdits fluides en estimant une quantité de chacune desdites classes chimiques du schéma compositionnel en appliquant le schéma réactionnel.

2. Méthode selon la revendication 1, dans laquelle lesdits accepteurs d'électrons sont choisis parmi les accepteurs suivants : $O_2$, $NO_3$, $SO_4$ et $H_2O$.

3. Méthode selon l'une des revendications précédentes, dans laquelle lesdites vitesses de réactions sont définies par un produit d'un premier terme ($R_t$) qui permet de prendre en compte un effet du temps de séjour dans le milieu poreux, un second terme ($R_T$) pour prendre en compte un effet de la température, et un troisième terme ($V_c$) pour prendre en compte l'accessibilité et la biodégradabilité intrinsèque de chacune des classes chimiques.

4. Méthode selon la revendication 3, dans laquelle le premier terme $R_t$ représente un rendement de biodégradation et est déterminé au moyen d'une première courbe d'évolution dudit rendement $R_t$ en fonction du temps de séjour dans le milieu.

5. Méthode selon la revendication 4, dans laquelle ladite première courbe est une fonction exponentielle du temps, où ledit rendement $R_t$ est supérieur à 95 % au-delà d'environ 2000 ans.

6. Méthode selon l'une des revendications 3 à 5, dans laquelle le second terme $R_T$ représente un rendement de biodégradation et est déterminé au moyen d'une courbe gaussienne d'évolution dudit rendement $R_T$ en fonction de la température dans le milieu.

7. Méthode selon la revendication 6, dans laquelle ladite courbe gaussienne est centrée sur une température d'environ 30°C et s'annule pour 0°C et environ 70°C.

**8.** Méthode selon l'une des revendications 3 à 7, dans laquelle le troisième terme ($V_c$) est déterminé en considérant que la vitesse de biodégradabilité intrinsèque des $C_6$-$C_{14}$ saturés est plus grande que celles des autres classes, et en considérant que la vitesse d'accessibilité maximale des $C_6$-$C_{14}$ aromatiques est plus grande que celles des autres classes.

**9.** Méthode selon l'une des revendications 3 à 8, dans laquelle lesdites vitesses de réactions sont définies par :

$$
\begin{array}{lcl}
V\ C_{14}\_sat & = & 0.4 x V_{max} \\
V\ C_{14}\_aro & = & 0.3 x V_{max} \\
V\ C_{14+}n & = & 0.2 x V_{max} \\
V\ C_{14+}iso & = & 0.18 x V_{max} \\
V\ C_{14+}cyclanes & = & 0.08 x V_{max} \\
V\ C_{14+}aro & = & 0.05 x V_{max} \\
V\ NSO & = & 0
\end{array}
$$

où $V_{max}$ est vitesse maximale de biodégradation définie par le produit de la vitesse maximale de biodégradabilité intrinsèque par la vitesse maximale d'accessibilité.

**10.** Méthode selon l'une des revendications précédentes, dans laquelle ledit milieu est réservoir pétrolier, et on déduit de ladite composition des fluides une quantité de gaz acides produit lors de la biodégradation, pour déterminer des conditions d'exploitation dudit réservoir pétrolier.

**11.** Méthode selon l'une des revendications précédentes, dans laquelle ledit milieu est réservoir pétrolier, et on déduit de ladite composition des fluides une quantité de méthane produit lors de la biodégradation, pour déterminer des conditions d'exploitation dudit réservoir pétrolier.

**Claims**

**1.** A method of determining a composition of fluids present in a porous medium after biodegradation, **characterized in that** it comprises:

- defining a biodegradation compositional scheme from at least the following chemical classes: $CO_2$, n-saturated $H_2S$, $C_1$, $C_2$-$C_4$, $C_6$-$C_{14}$ and iso-saturated $C_6$-$C_{14}$, cyclo-saturated $C_6$-$C_{14}$, $C_6$-$C_{14}$ aromatics, n-saturated $C_{14+}$, iso-saturated $C_{14+}$, cyclo-saturated $C_{14+}$, $C_{14+}$ aromatics, NSOs,
- determining an amount of each one of said chemical classes contained in said fluids before biodegradation,
- defining a biodegradation reaction scheme wherein said chemical classes react with electron acceptors and wherein it is imposed that said classes react simultaneously with a first electron acceptor having the highest oxydoreduction potential, according to different reaction velocities and up to depletion of said first acceptor in the medium, then they react sequentially towards the other electron acceptors according to the same principle,
- determining said reaction velocities of each one of said chemical classes according to at least: - the potential concentration of the chemical classes in the water; - their intrinsic biodegradability; - their reactivity to said electron acceptors; - the temperature of the medium; and - the residence time of said fluids within the medium, and
- determining the composition of said fluids by assessing an amount for each one of said chemical classes of the compositional scheme by applying the reaction scheme.

**2.** A method as claimed in claim 1, wherein said electron acceptors are selected from among the following acceptors: $O_2$, $NO_3$, $SO_4$ and $H_2O$.

**3.** A method as claimed in any one of the previous claims, wherein said reaction velocities are defined by a product of a first term ($R_t$) allowing an effect of the residence time in the porous medium to be taken into account, a second term ($R_T$) allowing an effect of temperature to be taken into account and a third term ($V_c$) allowing the accessibility and the intrinsic biodegradability of each chemical class to be taken into account.

**4.** A method as claimed in claim 3, wherein first term $R_t$ represents a biodegradation efficiency and it is determined by means of a first evolution curve of said efficiency $R_t$ as a function of the residence time in the medium.

**5.** A method as claimed in claim 4, wherein said first curve is an exponential function of time, where said efficiency $R_t$ is above 95 % beyond about 2000 years.

**6.** A method as claimed in any one of claims 3 to 5, wherein second term $R_T$ represents a biodegradation efficiency and it is determined by means of a Gaussian evolution curve of said efficiency $R_T$ as a function of the temperature in the medium.

**7.** A method as claimed in claim 6, wherein said Gaussian curve is centered on a temperature of about 30°C and it is equated to zero for 0°C and about 70°C.

**8.** A method as claimed in any one of claims 3 to 7, wherein third term $V_c$ is determined by considering that the intrinsic biodegradability velocity of the saturated $C_6$-$C_{14}$ is higher than that of the other classes, and by considering that the maximum accessibility velocity of the $C_6$-$C_{14}$ aromatics is higher than that of the other classes.

**9.** A method as claimed in any one of claims 3 to 8, wherein said reaction velocities are defined by:

$$
\begin{array}{lll}
V\ C_{14}\text{-sat} & = & 0.4XV_{maX} \\
V\ C_{14}\text{-aro} & = & 0.3xV_{max} \\
V\ C_{14+}n & = & 0.2xV_{max} \\
V\ C_{14+}\text{iso} & = & 0.18xV_{max} \\
V\ C_{14+}\text{cyclanes} & = & 0.08xV_{max} \\
V\ C_{14+}\text{aro} & = & 0.05xV_{max} \\
V\ NSO & = & 0
\end{array}
$$

where $V_{max}$ is the maximum biodegradation velocity defined by the product of the maximum intrinsic biodegradability velocity by the maximum accessibility velocity.

**10.** A method as claimed in any one of the previous claims, wherein said medium is a petroleum reservoir, and an amount of acid gas produced upon biodegradation is deduced from said composition of the fluids in order to determine development conditions for said petroleum reservoir.

**11.** A method as claimed in any one of the previous claims, wherein said medium is a petroleum reservoir, and an amount of methane produced upon biodegradation is deduced from the composition of the fluids in order to determine development conditions for said petroleum reservoir.

**Patentansprüche**

**1.** Verfahren zur Bestimmung der Zusammensetzung von in einem porösen Milieu anwesenden Fluiden nach einer Biodegradation, **gekennzeichnet durch** die Schritte:

- man definiert ein Biodegradationszusammensetzungsschema ausgehend von wenigstens den folgenden chemischen Klassen: $CO_2$, $H_2S$, $C_1$, $C_2$-$C_4$, gesättigten n-$C_6$-$C_{14}$, gesättigten iso-$C_6$-$C_{14}$, gesättigten cyclo-$C_6$-$C_{14}$, aromatische $C_6$-$C_{14}$, gesättigten n-$C_{14+}$, gesättigte iso-$C_{14+}$, gesättigte cyclo-$C_{14+}$, aromatische $C_{14+}$, $NSO_S$;
- man bestimmt eine Menge jeder der in den Fluiden vor der Biodegradation enthaltenen chemischen Klassen;
- man definiert ein Reaktionsschema der Biodegradation, in dem die chemischen Klassen mit Elektronenakzeptoren reagieren, und in dem man die chemischen Klassen simultan dazu zwingt, mit einem ersten Elektronenakzeptor zu reagieren, der das stärkste Redoxpotential gemäß der unterschiedlichen Reaktionsgeschwindigkeiten aufweist, bis zur Erschöpfung des ersten Akzeptors in dem Milieu, dann nacheinander mit anderen Elektronenakzeptoren nach demselben Prinzip zu reagieren;
- man bestimmt die jeweiligen Reaktionsgeschwindigkeiten der chemischen Klassen als Funktion von wenigstens: -der potentiellen Konzentration der jeweiligen chemischen Klasse im Wasser; -ihrer intrinsischen Biodegradationsfähigkeit; -ihrer Reaktivität im Verhältnis zu den Elektronenakzeptoren; -der Temperatur des Milieus; und -der Aufenthaltszeit der Fluide in dem Milieu; und
- man bestimmt die Zusammensetzung der Fluide **durch** Abschätzen einer Menge jeder der chemischen Klassen des Schemas der Zusammensetzung durch Anwenden des Reaktionsschemas.

**2.** Verfahren gemäß Anspruch 1, bei dem die Elektronenakzeptoren ausgewählt sind aus den folgenden Akzeptoren: $O_2$, $NO_3$, $SO_4$ und $H_2O$.

**3.** Verfahren nach einem der vorherigen Ansprüche, bei dem die Reaktionsgeschwindigkeiten definiert sind durch ein Produkt aus einem ersten Term ($R_t$), der es erlaubt, einen Effekt der Aufenthaltsdauer in dem porösen Milieu zu berücksichtigen, einem zweiten Term ($R_T$), der es erlaubt, einen Temperatureffekt zu berücksichtigen, und einem dritten Term ($V_c$), der die Zugänglichkeit und die intrinsische Biodegradationsfähigkeit jeder der chemischen Klassen berücksichtigt.

**4.** Verfahren gemäß Anspruch 3, bei dem der erst Term $R_t$ eine Ausbeute der Biodegradation darstellt und bestimmt wird mittels einer ersten Kurve der Ausbeute $R_t$ als Funktion der Aufenthaltszeit in dem Milieu.

**5.** Verfahren gemäß Anspruch 4, bei dem die erste Kurve eine exponentielle Funktion der Zeit ist, bei der die Ausbeute $R_t$ größer als 95% bei mehr als etwa 2000 Jahren ist.

**6.** Verfahren gemäß einem der Ansprüche 3 bis 5, bei dem der zweite Term $R_T$ eine Ausbeute der Biodegradation darstellt und bestimmt wird mittels einer Gaußkurve der Ausbeute $R_T$ als Funktion der Temperatur im Milieu.

**7.** Verfahren gemäß Anspruch 6, bei dem die Gaußkurve bei einer Temperatur von ungefähr 30°C zentriert ist und bei 0°C und ungefähr 70°C die Ordinate im Wesentlichen erreicht hat.

**8.** Verfahren gemäß einem der Ansprüche 3 bis 7, bei dem der dritte Term ($V_c$) durch Berücksichtigung der Tatsache bestimmt wird, daß die intrinsische Biodegradationsfähigkeit der gesättigten $C_6$-$C_{14}$ größer ist als diejenige der anderen Klassen, und durch Berücksichtigung der Tatsache, daß die maximale Geschwindigkeit der Zugänglichkeit der aromatischen $C_6$-$C_{14}$ größer ist als die der anderen Klassen.

**9.** Verfahren gemäß einem der Ansprüche 3 bis 8, bei dem die Reaktionsgeschwindigkeiten definiert sind durch:

$$V\, C_{14\text{-}}\text{ ges.} = 0,4 \times V_{max}$$

$$V\, C_{14\text{-}}\text{ arom.} = 0,3 \times V_{max}$$
$$V\, C_{14+}\, n = 0,2 \times V_{max}$$
$$V\, C_{14+}\, iso = 0,18 \times V_{max}$$
$$V\, C_{14+}\, cycl. = 0,08 \times V_{max}$$
$$V\, C_{14+}\, arom. = 0,05 \times V_{max}$$
$$V\, NSO = 0$$

wobei $V_{max}$ die maximale Geschwindigkeit der Biodegradation ist, definiert durch das Produkt aus der maximalen intrinsischen Biodegradationsfähigkeit mit der maximalen Geschwindigkeit der Zugänglichkeit.

**10.** Verfahren nach einem der vorherigen Ansprüche, bei dem das Milieu ein erdölgefülltes Reservoir ist und bei dem man aus der Zusammensetzung der Fluide eine Menge sauren Gases ableitet, das durch die Biodegradation erzeugt wurde, um die Explorationsbedingungen des erdölgefollten Reservoirs zu bestimmen.

**11.** Verfahren nach einem der vorherigen Ansprüche, bei dem das Milieu ein erdölgefülltes Reservoir ist und bei dem man aus der Zusammensetzung der Fluide eine Menge an Methan ableitet, das durch die Biodegradation erzeugt wurde, um die Explorationsbedingungen des erdölgefüllten Reservoirs zu bestimmen.

**Fig. 1**

**Fig. 2**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1436412 A
- FR 2888251 A
- FR 2906482

**Littérature non-brevet citée dans la description**

- **Conan et al.** Advances in Organic Geochemistry. Pegamon Press Oxford, 1979, 1-17
- **Peters ; Moldowan.** The biomarker guide. Prentice Hall, 1993
- **Larter et al.** *Organic Geochemistry,* 2003, vol. 4, 6001-613
- **Behar et al.** *Organic Geochemistry,* 2006, vol. 37, 1042-1051
- **de Barros Penteado et al.** *Organic Geochemistry,* 2007, vol. 38, 1197-1211